# EUROPÄISCHE PATENTSCHRIFT

(11) **EP 3 097 071 B1**
(45) Veröffentlichungstag und Bekanntmachung des Hinweises auf die Patenterteilung: **03.03.2021**
(21) Anmeldenummer: 15700453.2
(22) Anmeldetag: 14.01.2015
(51) Int. Cl.: C07C 15/04, C07C 15/06, C07C 5/10, C07C 5/11, C01B 3/00

(54) **VERFAHREN ZUR STOFFLICHEN NUTZUNG VON WASSERSTOFF**
METHOD FOR UTILIZING HYDROGEN
METHOD FOR UTILIZING HYDROGEN

(30) Priorität: 24.01.2014 DE 102014201332
(43) Veröffentlichungstag der Anmeldung: 30.11.2016
(73) Patentinhaber: HYDROGENIOUS LOHC TECHNOLOGIES GMBH, 91058 Erlangen (DE)
(72) Erfinder: ARLT, Wolfgang, 90425 Nürnberg (DE); BÖSMANN, Andreas, 91093 Hessdorf (DE); PREUSTER, Patrick, 91180 Heideck (DE); WASSERSCHEID, Peter, 91054 Erlangen (DE)
(74) Vertreter: Rau, Schneck & Hübner Patentanwälte Rechtsanwälte PartGmbB
(86) Internationale Anmeldenummer: PCT/EP2015/050604
(87) Internationale Veröffentlichungsnummer: WO 2015/110334

(56) Entgegenhaltungen:
- FR-A1- 2 862 630
- GB-A- 925 457
- GB-A- 1 152 383
- US-A- 6 074 447

## Beschreibung

Auf den Inhalt der deutschen Patentanmeldung DE 10 2014 201 332.1 wird vollumfänglich Bezug genommen.

Die Erfindung betrifft ein Verfahren zur stofflichen Nutzung von Wasserstoff, insbesondere aus flüssigen organischen Hydriden.

Eine stoffliche Nutzung von Wasserstoff beispielsweise durch die Verwendung von Wasserstoff als Rohstoff für eine katalytische Hydrierung organischer oder anorganischer Verbindungen ist wirtschaftlich von hoher Bedeutung. Die stoffliche Nutzung von Wasserstoff erfordert nach der FR 2 862 630 A1 und US 6,074,447 A üblicherweise die Handhabung von molekularem Wasserstoff. Die Verwendung von molekularem gasförmigen Wasserstoff ist nur unter hohem Druck möglich. Die Lagerung und Handhabung von molekularem Wasserstoff sind kompliziert und sicherheitstechnisch riskant. Um die sicherheitstechnischen Risiken zu minimieren, ist ein hoher apparativer und damit verbundener Kostenaufwand erforderlich. Insbesondere ist die Wasserstoffkompression ein energieintensiver Verfahrensschritt, der insbesondere die Verwendung von kosten- und wartungsintensiven Kompressoren erfordert.

Der vorliegenden Erfindung liegt die Aufgabe zugrunde, die Verfügbarkeit von Wasserstoff für dessen stoffliche Nutzung zu verbessern.

Die Aufgabe wird durch die Merkmale des Anspruchs 1 gelöst. Ein System zur stofflichen Nutzung von Wasserstoff weist eine Transferhydrier-Einrichtung mit einer Transferhydrier-Einheit zum Hydrieren eines Hydrierguts wie beispielsweise eines ungesättigten organischen Moleküls, insbesondere Toluol, auf. Als stoffliche Nutzung wird die Nutzung von Wasserstoff allgemein in Hydrierreaktionen der chemischen und/oder der stoffumwandelnden Industrie verstanden. Derartige Hydrierreaktionen dienen der Umsetzung organischer oder anorganischer chemischer Verbindungen mit Wasserstoff zu Verbindungen, deren Wasserstoffgehalt gegenüber der Ausgangsverbindung oder den Ausgangsverbindungen erhöht ist. Bei der stofflichen Verwendung von Wasserstoff in einer Hydrierreaktion wird Wasserstoff genutzt, um unter Aufbau neuer chemischer Bindungen den Wasserstoffgehalt des Hydrierguts zu erhöhen. Zum Bereitstellen von Wasserstoff für die Transferhydrier-Einrichtung ist erfindungsgemäß eine Wasserstoffbereitstellungs-Einrichtung vorgesehen. Es ist insbesondere vorgesehen, eine wasserstoffreiche Verbindung bereitzustellen, die für die Verwendung in der Transferhydrier-Einrichtung insbesondere aus einer wasserstoffarmen Verbindung durch Hydrieren hergestellt worden ist. Mittels der Wasserstoffbereitstellungs-Einrichtung wird Wasserstoff in gebundener Form, insbesondere in chemisch gebundener Form, für die Transferhydrier-Einrichtung bereitgestellt. Die Wasserstoffbereitstellungs-Einrichtung weist eine Belade-Einheit zum Beladen eines Trägermediums mit Wasserstoff auf. Eine derartige Belade-Einheit ermöglicht unkompliziert und vergleichsweise unaufwändig die chemische Bindung des Wasserstoffs an das Trägermedium, insbesondere an LOHC. Diese Form der Wasserstoffspeicherung hat den besonderen Vorteil, dass LOHC-Trägermedien unter den verwendeten Prozessbedingungen als organische Verbindung in flüssiger Form vorliegen. Insbesondere ermöglichen die LOHC-Trägermedien, dass sie reversibel mit Wasserstoff beladen und von Wasserstoff entladen werden können. Die physikochemischen Eigenschaften der LOHC-Trägermedien haben hohe Ähnlichkeit zu herkömmlichen flüssigen Kraftstoffen, sodass Pumpen und Tankfahrzeuge zum Transport und Behälter zur Lagerung aus dem Bereich der Kraftstoff- und Brennstofflogistik genutzt werden können. Die Wasserstoffspeicherung in chemisch gebundener Form in einer organischen Flüssigkeit erlaubt eine drucklose Lagerung bei Normalbedingungen über große Zeiträume ohne signifikanten Wasserstoffverlust. Als LOHC-Trägermedien sind insbesondere polyzyklische, aromatische Verbindungen mit einem π-Elektronensystem oder mehreren π-Elektronensystemen nutzbar, die in einer katalytischen Hydrierung in die jeweiligen gesättigten, polyzyklischen Verbindungen überführt werden. Als LOHC-Trägermedien können insbesondere Dibenzyltoluole und Benzyltoluole als Reinstoffe, isomere Gemische oder Mischungen dieser Substanzen miteinander verwendet werden. Es ist auch möglich, als LOHC-Trägermedien polyzyklische, heteroaromatische Verbindungen mit einem π-Elektronensystem oder mehreren π-Elektronensystemen zu verwenden, die in der Belade-Einheit durch Hydrierung in die jeweiligen gesättigten, polyzyklischen Verbindungen überführt werden, die Heteroatome wie Stickstoff oder Sauerstoff enthalten. Insbesondere dienen als LOHC-Trägermedien N-Ethylcarbazol, N-Propylcarbazol, N-Isopropylcarbazol, N-Butylcarbazol oder Mischungen dieser Substanzen miteinander. Als LOHC-Trägermedien sind organische Oligomere oder Polymere mit ausgedehnten π-konjugierten Elektronensystemen, die in der Belade-Einheit durch Hydrierung in die jeweiligen gesättigten Verbindungen überführt werden, möglich. Das Hydrieren der wasserstoffentladenen Form der LOHC-Trägermedien erfolgt in einem druckstabilen chemischen Reaktor bei einer Temperatur zwischen 50°C und 400°C, insbesondere zwischen 120°C und 300°C, insbesondere zwischen 150°C und 280°C. Die Hydrierung, also das Beladen des LOHC-Trägermediums, findet bei einem Verfahrensdruck von 2 bar bis 200 bar, insbesondere bei 10 bar bis 100 bar und insbesondere in Gegenwart eines metallhaltigen Katalysators statt. Als Katalysatoren für die Beladung des LOHC-Trägermediums eignen sich insbesondere solche, die das Element Ruthenium und/oder Nickel aufweisen. Es sind auch Katalysatoren möglich, die andere Elemente oder zusätzliche Elemente neben Ruthenium und/oder Nickel aufweisen. Wesentlich sind solche Elemente, die Wasserstoff anlagern und auf LOHC-Trägermedium übertragen können. Neben Ruthenium und/oder Nickel sind insbesondere Metalle wie Chrom, Eisen, Kobalt, Kupfer, Iridium, Palladium oder Platin als Katalysatoren möglich. Das mit Wasserstoff-beladene LOHC Trägermedium stellt eine geeignete Transportform und Speicherform für den chemisch gebundenen Wasserstoff dar, da die physikalisch-chemischen Eigenschaften des Wasserstoffbeladene LOHC Trägermedium denen von Diesel und anderen Kraftstoffen stark ähneln.

Nach dem Stand der Technik wird ein mit Wasserstoff-beladene LOHC-Trägermedium am Ort und zur Zeit des Energiebedarfs in einer Entlade-Einheit unter Zuführung von Wärme und in Gegenwart eines geeigneten Katalysators unter Freisetzung von molekularem Wasserstoff wieder in die Wasserstoff-entladene Form überführt. Bei der Entladung von LOHC-Trägermaterialien wird der Wasserstoff durch eine katalysierte Dehydrierreaktion aus einem organischen Molekül oder aus einer Mischung organischer Moleküle freigesetzt. Das bedeutet, dass die Freisetzung des Wasserstoffs durch eine stoffliche Umwandlung des beladenen Trägermediums durch Entladung in der Entlade-Einheit mittels katalysierter Dehydrierreaktion erfolgt. Im beladenen Zustand ist das Trägermedium insbesondere eine gesättigte, polyzyklische Verbindung, insbesondere ein Perhydro-Dibenzyltoluol oder ein Perhydro-Benzyltoluol, die als Reinstoffe, isomere Gemische oder Mischungen untereinander verwendet werden können. Alternativ ist das beladene Trägermedium eine gesättigte, polyzyklische Verbindung, die Heteroatome wie Stickstoff oder Sauerstoff enthalten, insbesondere Perhydro-N-Ethylcarbazol, Perhydro-N-Propylcarbazol, Perhydro-N-Isopropylcarbazol, Perhydro-N-Butylcarbazol oder Mischungen dieser Substanzen. Alternativ können als beladenes Trägermedium auch ein gesättigtes organisches Oligomer oder Polymer verwendet werden, die sich durch katalytische Dehydrierung in Oligomere oder Polymere mit ausgedehntem π-konjugierten Elektronensystem umsetzen lassen. Das Entladen der beladenen Trägermedien in der Entlade-Einheit erfolgt insbesondere in einem druckstabilen chemischen Reaktor bei einer Prozesstemperatur zwischen 100° C und 450° C, bevorzugt zwischen 150° C und 420°C und insbesondere zwischen 180° C und 390° C. Der Prozessdruck liegt zwischen 0,1 und 30 bar, insbesondere zwischen 1 und 10 bar, wobei insbesondere ein metallhaltiger Katalysator eingesetzt werden kann, der insbesondere Platin und/oder Palladium enthält. Wesentlich ist, dass der Katalysator geeignet ist, Wasserstoff, der vom LOHC-Trägermedium abgegeben wird, als Wasserstoffgas freizusetzen. Neben Platin und/oder Palladium sind dafür insbesondere Metalle wie Chrom, Eisen, Kobalt, Nickel, Kupfer, Iridium oder Ruthenium, geeignet.

Dadurch, dass Wasserstoff in gebundener Form für die stoffliche Nutzung zum Hydrieren des Hydrierguts bereitgestellt wird, entfällt die Lagerung und Handhabung von molekularem Wasserstoff. Mittels dem System kann die Gewinnung von molekularem Wasserstoff aus einem wasserstoffbeladenen Trägermedium in einem separaten Verfahrensschritt entfallen. Stattdessen wird das flüssige, wasserstoffbeladene Trägermedium in der Transferhydrier-Einrichtung direkt mit einem Hydriergut kontaktiert. Dabei wird Wasserstoff von dem beladenen Trägermedium auf das Hydriergut übertragen. Die mit der Lagerung und Handhabung von molekularem Wasserstoff verbundenen Nachteile entfallen ebenfalls. Als besonders vorteilhaft hat sich die Verwendung von flüssigem organischen Hydrid als Trägermedium für die chemische Bindung von Wasserstoff erwiesen. Ein derartiges Trägermedium ist an sich aus der EP 1 475 349 A2 bekannt und wird als Liquid Organic Hydrogen Carrier (LOHC) bezeichnet. Diese Form der Wasserstoffspeicherung ist beispielsweise aus dem Bereich der Handhabung und insbesondere Speicherung elektrischer Energie bekannt. Überraschend wurde nun gefunden, dass die Wasserstoffspeicherung mittels eines Trägermediums, insbesondere mittels LOHC-Trägermedien, insbesondere in flüssiger Form, zur stofflichen Nutzung von Wasserstoff, insbesondere zur Hydrierung, vorteilhaft genutzt werden kann. Gegenüber der direkten energetischen Nutzung beispielsweise in einer Brennstoffzelle oder in einer mit Wasserstoff befeuerten Brennkammer ergibt sich für die stoffliche Nutzung von Wasserstoff, insbesondere zur Hydrierung organischer oder anorganischer Moleküle, ein zusätzlicher Vorteil gemäß der Erfindung dadurch, dass eine der stofflichen Nutzung vorgeschaltete Entladung des Trägermediums nicht erforderlich ist. Es ist also nicht erforderlich, dass der chemisch gebundene Wasserstoff von dem Trägermedium in einem separaten Verfahrensschritt getrennt werden muss, um für die nachfolgende stoffliche Nutzung von Wasserstoff weiter verwendet werden zu können. Erfindungsgemäß wurde also erkannt, dass der Wasserstoff in der gebundenen Form für die stoffliche Nutzung in der Transferhydrier-Einrichtung direkt verwendet werden kann. Zudem wurde gefunden, dass die aus der Verwendung von LOHC-Trägermedien resultierenden Vorteile auf die Handhabung und Speicherung von Wasserstoff für die stoffliche Nutzung von Wasserstoff übertragbar sind.

Vorteilhaft ist ein System, bei dem in der Transferhydrier-Einheit ein Katalysator vorgesehen ist, um das Hydrieren durch eine Kontaktier-Einheit zum Kontaktieren des Hydrierguts mit dem in gebundener Form bereitgestellten Wasserstoff zu fördern. In der Transferhydrier-Einheit wird das Hydriergut mit dem gebundenen Wasserstoff kontaktiert. Insbesondere kann das Hydriergut in fester, flüssiger oder gasförmiger Form vorliegen, wenn es mit dem gebundenen Wasserstoff kontaktiert wird. Die Kontaktierung des Hydrierguts mit dem in gebundener Form bereitgestellten Wasserstoff erfolgt also in Gegenwart eines Katalysators. Insbesondere dient dazu ein fester Katalysator, also ein heterogener Katalysator. Ein derartiger Katalysator ermöglicht die Katalyse der Wasserstofffreisetzung aus dem beladenen flüssigen organischen Hydrid und/oder die Hydrierung des Hydrierguts selbst. Als geeignete Katalysatoren haben sich insbesondere Metalle erwiesen, die mindestens eines der Elemente Platin, Palladium, Chrom, Eisen, Nickel, Kobalt, Kupfer, Iridium oder Ruthenium aufweisen. Wesentlich ist dabei, dass der eingesetzte Katalysator geeignet ist, Wasserstoff, der vom Trägermedium, insbesondere vom LOHC-Trägermedium abgegeben wird, als Wasserstoffgas freizusetzen, das freigesetzte Wasserstoffgas am Hydriergut chemisch anzulagern und/oder den an der Katalysatoroberfläche gebundenen Wasserstoff auf das Hydriergut zu übertragen. Es sind insbesondere auch Katalysatoren möglich, die mehr als eines der genannten Metalle auf einem Trägermaterial verwenden. Es ist ferner auch möglich, mehr als einen festen Katalysator in Mischungen für die Transferhydrierung einsetzen, wobei mindestens einer der eingesetzten Katalysatoren mindestens eines der genannten Metalle enthält. Ein weiterer wesentlicher Vorteil besteht darin, dass die bei der Hydrierung freigesetzte Reaktionswärme im Wesentlichen der für die Wasserstofffreisetzung aus dem beladenen flüssigen organischen Hydrid aufzubringenden Wärme entspricht. Das bedeutet, dass eine hohe Reaktionswärme der Hydrierung nicht mit aufwändigen Kühleinrichtungen aus einem Reaktor abgeführt werden muss. Derartige Kühlprobleme sind aus dem Stand der Technik bekannt und führen - wenn eine Kühlung durch aufwändige Kühleinrichtungen nicht in ausreichender Form erfolgt - zu einer Überhitzung des Hydrierguts und des Katalysators. Derartige Überhitzungen können zu Selektivitätsproblemen der Hydrierreaktion einerseits und zu Lebensdauerbeeinträchtigungen des eingesetzten Katalysators andererseits führen. Zudem kann eine unkontrollierte Überhitzung zu einer spontanen Zersetzung des Hydrierguts führen. Damit sind in der Regel erhebliche sicherheitstechnische Probleme verbunden. Darüber hinaus muss Wärme nicht zusätzlich bereitgestellt werden, um die Entladung des wasserstoffbeladenen Trägermediums zu ermöglichen. Die hierfür erforderliche Wärme kann direkt, zumindest zu einem großen Anteil, der Reaktionswärme des Hydriervorgangs entnommen werden. Eine aufwändige Kühlung oder Heizung der Transferhydrier-Einheit kann entfallen.

Besonders vorteilhaft ist ein System, bei der die Transferhydrier-Einrichtung eine Stofftrennungs-Einheit aufweist. Dadurch ist es möglich, mit dem System einen Hybridprozess durchzuführen. Ein derartiger Hybridprozess wird beispielsweise bei einer Veresterung angewendet. Das System ermöglicht neben der Hydrierfunktion zusätzlich die Funktion einer destillativen Stofftrennung. Die so gestaltete Transferhydrier-Einrichtung dient als Reaktivdestillations-Einheit, die zum Trennen hydrierter und nicht-hydrierter bzw. nicht vollständig hydrierter Bestandteile des Hydrierguts dient. Überraschenderweise wurde gefunden, dass das als Trägermedium für den Wasserstoff eingesetzte flüssige organische Hydrid zusätzlich zu der wasserstoffspendenden Wirkung auch eine Wirkung eines Extraktivlösemittels oder eines Entrainers im Rahmen dieser Reaktivdestillation hat. Damit ist das Trennergebnis von hydrierten und nicht-hydrierten bzw. teilhydrierten Bestandteilen des Hydrierguts verbessert. Für die Beurteilung des Trennergebnisses ist es ausreichend, wenn Trennfaktoren hydrierter und nicht-hydrierter bzw. nicht vollständig hydrierter Bestandteile des Hydrierguts unter dem Einfluss des flüssigen organischen Hydrids verglichen werden. Eine mögliche Messmethode ist aus dem Stand der Technik als inverse Gaschromatographie bekannt. Diese Messmethode ist in G. Sadowski et al.: Fluid Phase Equilib. 139 (1997), 391 bis 403 beschrieben. Dort wurde das Stoffgemisch Methylcyclohexan-Toluol der Untersuchung zugrunde gelegt. Der Trennfaktor ohne Entrainer liegt bei 1,5. Mit der genannten Messmethode wurde gefunden, dass der Trennfaktor unter dem Einfluss des unhydrierten organischen Hydrids N-Ethylcarbazol temperaturabhängig bestimmt werden kann. Bei einer Messtemperatur von 70,76°C beträgt der Trennfaktor 3,41, bei einer Messtemperatur von 88,64°C beträgt der Trennfaktor 3,01 und einer Messtemperatur von 110,11 °C beträgt der Trennfaktor 2,64. Vorteilhaft bei dieser Ausführungsform ist also, dass die Trennung von Methylcyclohexan von Toluol in einer kürzeren Stofftrennungs-Einheit verglichen mit fehlender Extraktiv-Rektifikationswirkung möglich ist. Insbesondere ist auch eine Reduktion des Energiebedarfs durch Verringerung des Rücklaufverhältnisses möglich. Damit kann der Hybridprozess, insbesondere eine Reaktiv-Extraktiv-Rektifikation, vorteilhaft umgesetzt werden.

Besonders vorteilhaft ist ein System, bei dem die Transferhydrier-Einheit ein Rührwerk aufweist. Insbesondere ist die Transferhydrier-Einheit als Druckbehälter ausgeführt, in dem der Katalysator mittels des Rührwerks mit dem wasserstoffbeladenen Trägermedium, insbesondere dem wasserstoffbeladenen LOHC-Trägermedium und dem Hydriergut intensiv kontaktiert wird. Alternativ kann die Kontaktierung auch durch vermischendes Pumpen mittels einer Pumpe oder durch Fluid-Anströmung mittels einer Anström-Einheit erfolgen. Die Transferhydrier-Einheit enthält unabhängig von der Wahl der Kontaktierung insbesondere eine bewegte Suspension, in der fester Katalysator fein verteilt, das flüssige Trägermedium und das Hydriergut vorliegen.

Vorteilhaft ist ein System, bei dem die Wasserstoffbereitstellungs-Einrichtung eine Trägermediumspeicherungs-Einheit zum Speichern des beladenen Trägermediums aufweist. Dadurch ist es möglich, zu einem beliebigen Zeitpunkt, insbesondere unabhängig von dem tatsächlichen Bedarf des beladenen Trägermediums, das beladene Trägermedium zu speichern, also zwischenzulagern. Mit der Trägermediumspeicherungs-Einheit kann das beladene Trägermedium verlustfrei über einen längeren Zeitraum gespeichert werden. Das beladene Trägermedium kann aus der Trägermediumspeicherungs-Einheit, die insbesondere als Flüssigkeitstank ausgeführt ist, zu einem erforderlichen Zeitpunkt, also dann, wenn die stoffliche Nutzung des Wasserstoffs erfolgen soll, freigesetzt werden. Als Trägermediumspeicherungs-Einheit im Sinne der vorliegenden Patentanmeldung dienen auch geeignete Leitungen und Leitungssysteme und/oder TankFahrzeuge. Somit ist es möglich, das Trägermedium mit Wasserstoff in eine Wasserstoffbereitstellungs-Einrichtung mittels einer Belade-Einheit zu beladen, wobei das Beladen an einem ersten Ort erfolgen kann. Das beladene Trägermedium wird in der Trägermediumspeicherungs-Einheit gespeichert. Es ist möglich, das beladene Trägermedium beispielsweise mit dem Speicher zusammen in Form eines Tankfahrzeugs von dem ersten Ort der Beladung zu einem zweiten, von dem ersten Ort beabstandeten Ort zu transportieren. Das beladene Trägermedium kann an einem zweiten Ort, an dem selbst eine Belade-Einheit nicht zur Verfügung steht, zur Verfügung gestellt werden. Insbesondere kann ein derartiger Transport auch über ein entsprechendes Leitungsnetz erfolgen. Die Verfügbarkeit von beladenem Trägermedium ist dadurch zusätzlich verbessert.

Vorteilhaft ist ein System, bei dem die Wasserstoffbereitstellungs-Einrichtung eine Wasserstofferzeugungs-Einheit zum Erzeugen von Wasserstoff aufweist. Insbesondere dient die Wasserstofferzeugungs-Einheit zum Erzeugen von elementarem Wasserstoff. Die Wasserstofferzeugungs-Einheit ist insbesondere ein Elektrolyseur. In dem Elektrolyseur wird mittels Elektrolyse Wasser in Wasserstoff und Sauerstoff aufgespalten. Besonders vorteilhaft ist, wenn der für die Elektrolyse erforderliche elektrische Strom aus regenerativen Energieformen erzeugt worden ist wie beispielsweise mittels Photovoltaik-Anlagen, Windkraftwerken, Geothermiekraftwerken oder Wasserkraftwerken. Die Verfügbarkeit regenerativer Energien ist in Mitteleuropa hauptsächlich durch Wind- und Solarkraftwerke gegeben. Diese Energieformen sind von meteorologischen Einflüssen abhängig und insbesondere nicht beeinflussbar und nur schwer vorhersagbar. In bestimmten Regionen der Welt stehen regenerative Energieformen in großer Menge zur Verfügung. Dort ist die Stromerzeugung aus regenerativen Energien kostengünstig möglich. Dies gilt beispielsweise für die Stromerzeugung durch Geothermie in Island oder für die Stromerzeugung durch Wasserkraft in Norwegen und/oder Kanada oder für die Stromerzeugung durch Solarkraftwerke in sehr sonnenreichen Regionen der Erde. Um derart kostengünstig erzeugten elektrischen Strom von möglicherweise entlegenen Orten zu dem Ort des elektrischen Strombedarfs zu transportieren, kann an dem Ort der Stromerzeugung zusätzlich eine Belade-Einheit verwendet werden, um den elektrischen Strom in Form des beladenen Trägermediums in Versorgungsleitungen und/oder Transportfahrzeugen zu dem Ort des Strombedarfs, also zu dem Elektrolyseur für die Wasserstoffbereitstellungs-Einrichtung der Anlage zu transportieren. Als Elektrolyseur dient beispielsweise ein so bezeichneter Solid Oxide Electrolysis Cell (SOEC-)Elektrolyseur oder ein Polymer Elektrolyte Membran (PEM-)Elektrolyseur.

Vorteilhaft ist ein System mit einer mit der Transferhydrier-Einrichtung verbundenen Trenn-Einrichtung. Insbesondere ist die Trenn-Einrichtung mit einer Transferhydrier-Einheit der Transferhydrier-Einrichtung verbunden. Die Trenn-Einrichtung dient dem Trennen einer Mischung aus zumindest teilweise entladenem Trägermedium und zumindest teilweise hydriertem Hydriergut. Die Trenn-Einrichtung kann der Transferhydrier-Einrichtung nachgeschaltet sein. Es ist alternativ möglich, die Trenn-Einrichtung für eine Reaktivdestillation in die Transferhydrier-Einrichtung und insbesondere in die Transferhydrier-Einheit, zu integrieren. Die mittels der Trenn-Einrichtung abgetrennte, zumindest teilweise dehydrierte Form des flüssigen organischen Hydrids wird bevorzugt in einen Speicher, insbesondere in einen Lagertank, gepumpt und dort gelagert. Die Lagerung kann beispielsweise bis zu einem energiereichen Zeitraum andauern. Während eines energiereichen Zeitraums kann das entladene Trägermedium, insbesondere mit regenerativ erzeugtem, Wasserstoff erneut beladen werden. Energiereich bedeutet, dass mehr Energie, insbesondere aus regenerativen Energieformen erzeugter elektrischer Strom, zur Verfügung steht als verbraucht wird. Das bedeutet, es liegt ein Energieüberschuss vor. Die überschüssige Energie kann als Wasserstoff an dem Trägermedium gespeichert werden. Energiereich kann aber auch bedeuten, dass Energie in Form von elektrischem Strom zu vergleichsweise niedrigen Kosten verfügbar ist. Dagegen ist ein energiearmer Zeitraum dadurch gekennzeichnet, dass Energie nicht in ausreichender Menge oder nur zu hohen Kosten zur Verfügung steht. Das bedeutet, es wird mehr Energie benötigt, als zur Verfügung steht. Alternativ kann die zumindest teilweise dehydrierte Form des Trägermediums mit einem geeigneten Transportmittel an einen energiereichen Ort transportiert werden. Dort kann das entladene Trägermedium mit, insbesondere regenerativ erzeugtem, Wasserstoff erneut beladen werden, um zu einem späteren, insbesondere energiearmen Zeitpunkt zur stofflichen Nutzung des Wasserstoffs verwendet zu werden.

Besonders vorteilhaft ist die Ausführung der Trenn-Einrichtung als Destillations-Einrichtung. Alternativ kann die Trenn-Einrichtung auch als flüssig-flüssig-Phasentrennungs-Einrichtung oder als Extraktions-Einrichtung ausgeführt sein.

Vorteilhaft ist ein System, bei dem die Transferhydrier-Einheit mindestens einen Sensor, insbesondere einen Temperatur-Sensor und/oder einen Drucksensor, zur Überwachung von mindestens einer Prozessgröße aufweist. Als Prozessgröße dienen beispielsweise die Reaktionstemperatur in der Transferhydrier-Einheit oder der Innendruck in der Transferhydrier-Einheit. Insbesondere ist der mindestens eine Sensor mit einer Anzeige-Einheit in Signalverbindung, um die mindestens eine überwachte Prozessgröße anzuzeigen. Ein Bediener der Anlage kann in Abhängigkeit der angezeigten Prozessgröße auf das Verfahren einwirken und insbesondere die Prozessbedingungen derart verändern, dass vorgegebene Grenzwerte eingehalten werden. Beispielsweise ist es dadurch möglich, die Geschwindigkeit der Transferhydrierung zu beeinflussen und insbesondere gemäß einer Vorgabe zu steuern. Insbesondere bei einem Einsatz von temperaturempfindlichem Hydriergut ist es erforderlich, die Reaktionstemperatur in der Transferhydrier-Einheit zu begrenzen. Dadurch kann vermieden werden, dass eine thermisch induzierte Zersetzung des Hydrierguts einsetzt.

Vorteilhaft ist ein System mit einer Regelungs-Einheit zum Regeln mindestens einer Prozessgröße, insbesondere während der Transferhydrierung in der Transferhydrier-Einheit, wobei die Regelungs-Einheit mit mindestens einem Sensor, insbesondere mit einem Temperatur-Sensor und/oder mit einem Drucksensor, zur Überwachung der mindestens einen Prozessgröße in Signalverbindung steht. Die Signalverbindung kann kabelgebunden oder kabellos erfolgen. Insbesondere ist es denkbar, dass die Regelungs-Einheit dezentral und insbesondere beabstandet von der Anlage angeordnet ist. Eine Regelungs-Einheit kann beispielsweise in einer zentralen Überwachungseinrichtung angeordnet sein, die über mehrere km und insbesondere über mehrere hundert km, entfernt von der Anlage, insbesondere von der Transferhydrier-Einheit, angeordnet ist. Die mittels des mindestens einen Sensors erfassten Prozessgrößen werden an die Regelungs-Einheit übermittelt und dienen als Eingangsgrößen für eine Regelung der Prozessbedingungen. Die Regelungs-Einheit weist einen Regler auf, der erforderliche Stellsignale erzeugt, die über entsprechende Signalverbindungen an verschiedene Komponenten der Anlage übermittelt werden können. Dazu steht die Regelungs-Einheit beispielsweise mit einer Stromquelle, also einer Einrichtung zum Erzeugen von elektrischem Strom, insbesondere aus regenerativen Energieformen, mit dem Elektrolyseur, mit der Belade-Einheit und/oder mit der Trägermediumspeicherungs-Einheit in Signalverbindung. Insbesondere die Verbindung mit der Trägermediumspeicherungs-Einheit dient zur gesteuerten Zufuhr von beladenem Trägermedium in die Transferhydrier-Einheit. Um eine unerwünschte Überhitzung des Hydrierguts zu vermeiden, kann die Transferhydrier-Einheit mit einer Kühl-Einheit beispielsweise in Form eines Wärmetauschers verbunden sein. In diesem Fall ist die Regelungs-Einheit mit der Kühl-Einheit in Signalverbindung, um eine gezielte Wärmeabfuhr aus der Transferhydrier-Einheit zu veranlassen.

Vorteilhaft ist ein System, bei dem die Wasserstoffbereitstellungs-Einrichtung an einem energiereichen Ort angeordnet ist und die Transferhydrier-Einrichtung an einem von dem energiereichen Ort verschiedenen energiearmen Ort angeordnet ist. Ein derartiges System ermöglicht insbesondere das gezielte Ausnutzen von spezifischen Standortvorteilen. Als energiereicher Ort wird demnach ein Standort verstanden, bei dem elektrischer Strom, insbesondere kostengünstig aus regenerativen Energieformen wie beispielsweise Geothermie und/oder Wasserkraft, erzeugt werden kann. Derartige energiereiche Standorte sind beispielsweise Island oder Kanada. Der aus den regenerativen Energieformen erzeugte elektrische Strom wird an dem energiereichen Ort zur Elektrolyse genutzt, um Wasserstoff zu erzeugen und am energiereichen Ort zur Hydrierung eines Trägermediums, insbesondere eines LOHC-Trägermediums, zu verwenden. Das bedeutet, dass das Beladen des Trägermediums mit Wasserstoff mittels der Belade-Einheit an dem energiereichen Ort erfolgt. Dadurch, dass das wasserstoffbeladene Trägermedium besonders unkompliziert transportiert und/oder gespeichert werden kann, ist es möglich, den gebundenen Wasserstoff entsprechend vorteilhaft von dem energiereichen Ort zu dem energiearmen Ort zu transportieren, insbesondere in Tankfahrzeugen zu Lande und/oder zu Wasser. Vorteilhafterweise kann der gebundene Wasserstoff direkt in der Transferhydrier-Einrichtung mit dem Hydriergut umgesetzt werden. Es ist insbesondere denkbar, dass das Hydriergut nach erfolgter Hydrierung von dem entladenen Trägermedium isoliert wird, sodass das zumindest teilweise dehydrierte Trägermedium von dem energiearmen Ort, an dem die Hydrierung des Hydrierguts stattfindet, an den energiereichen Ort der Wasserstoffproduktion zurücktransportiert werden kann.

Besonders vorteilhaft ist ein System mit einer Transport-Einheit zum Transportieren des mittels der Wasserstoffbereitstellungs-Einrichtung in gebundener Form bereitgestellten Wasserstoffs von dem energiereichen Ort zu der Transferhydrier-Einrichtung an dem energiearmen Ort. Als Transportmittel werden beispielsweise Tankfahrzeuge wie Schiffe, Züge oder Lastkraftwagen verstanden. Als Transportmittel wird auch eine Förderleitung verstanden, mittels der das beladene Trägermedium direkt von dem energiereichen Ort zu dem energiearmen Ort transportiert werden kann. Gerade wegen der erheblichen Transportvorteile von trägermediumgebundenem Wasserstoff im Vergleich zum Transport von molekularem Wasserstoff kann sich die Belade-Einheit zum Beladen des Trägermediums mit Wasserstoff der Wasserstoffbereitstellungs-Einrichtung des Systems auch räumlich entfernt von der Transferhydrier-Einrichtung des Systems befinden. Vorteilhaft befindet sich die Belade-Einheit dort, wo aus regenerativen Energiequellen stammende Elektrizität besonders günstig in Wasserstoff umgewandelt werden kann. Die Transferhydrier-Einrichtung des Systems ist insbesondere dort vorgesehen, wo die Infrastruktur beispielsweise einer großen Chemieanlage die Herstellung, Lagerung und Weiterverarbeitung des Hydrierguts besonders geeignet ermöglicht. Der Transportweg zwischen der Belade-Einheit und der Transferhydrier-Einheit kann beispielsweise mehrere Tausend Kilometer betragen, wenn beispielsweise ein globaler Schifftransport des wasserstoffbeladenen Trägermediums der Transferhydrierung vorausgeht. Alternativ kann eine Pipeline-Verbindung oder ein Transport über Straße und/oder Schiene geeigneter sein, um das flüssige, wasserstoffbeladene Trägermedium von der Belade-Einheit zur Transferhydrier-Einheit zu bringen.

Das erfindungsgemäße Verfahren zur stofflichen Nutzung von Wasserstoff zeichnet sich vor allem dadurch aus, dass Wasserstoff in gebundener Form mittels einer Wasserstoffbereitstellungs-Einrichtung in Form eines flüssigen wasserstoffbeladenen Trägermediums bereitgestellt wird. Die Bindung des Wasserstoffs an das Trägermedium erfolgt erfindungsgemäß in einem vorgelagerten Belade-Schritt mittels einer Belade-Einheit. Der so, insbesondere chemisch, gebundene Wasserstoff kann zum Hydrieren eines Hydrierguts mittels einer Transferhydrier-Einheit einer Transferhydrier-Einrichtung genutzt werden. Insbesondere ist es also nicht erforderlich, dass Wasserstoff in elementarer Form gelagert und/oder gehandhabt werden muss. Zudem ist ein zusätzlicher Verfahrensschritt des Entladens eines mit Wasserstoff beladenen Trägermediums nicht erforderlich, da der Wasserstoff in der an dem Trägermedium gebundenen Form direkt zum Hydrieren eingesetzt werden kann.

Das erfindungsgemäße Verfahren kann zur Hydrierung unterschiedlicher Hydriergüter in der chemischen und petrochemischen Industrie, aber auch in anderen Industrien eingesetzt werden, die Hydrierreaktionen nutzen. Durch das System können organische oder anorganische chemische Verbindungen als Hydriergüter verwendet werden. Während der erfindungsgemäßen Transferhydrierung wird der Wasserstoffgehalt des Hydrierguts erhöht, wobei der dabei umgesetzte Wasserstoff dem System nicht als molekularer Wasserstoff, sondern gebunden an ein flüssiges, wasserstoffbeladenes Trägermedium zugeführt wird. Durch die Transferhydrierung wird eine wasserstoffarme Form des Hydrierguts in eine wasserstoffreiche Form des Hydrierguts überführt, wobei die wasserstoffreiche Form von höherem industriellem Wert ist. Typische wasserstoffarme Formen geeigneter Hydriergüter sind anorganische und organische Moleküle, die durch chemische Reaktion mit Wasserstoff neue chemische Bindungen aufbauen können. Ferner sind alle mit Wasserstoff reduzierbaren Verbindungen, insbesondere einfach und mehrfach ungesättigte organische Verbindungen wie beispielsweise Alkene und Alkine, aromatische Verbindungen, Verbindungen mit Heteroatom-Kohlenstoff-Doppelbindungen, wie beispielsweise Carbonylverbindungen, organische Säuren, Amide, Nitrile, Nitroverbindungen, sowie CO, CO₂ und N₂ geeignete Hydriergüter. Eine weitere Klasse an bevorzugten Hydriergütern sind heteroatomhaltige organische Verbindungen, bei denen durch Reaktion mit Wasserstoff das Heteroatom aus der organischen Verbindung herausgelöst wird. Dies sind beispielsweise organische Schwefelverbindungen, die in Kontakt mit Wasserstoff Schwefelwasserstoff und eine Verbindung bilden, die gegenüber der Ausgangsverbindung entschwefelt vorliegt. Besonders bevorzugt sind solche Hydriergüter, die in hydrierter Form einen Siedepunkt von 360 °C bei Normaldruck nicht überschreiten.

Besonders vorteilhaft ist ein Verfahren, bei dem ein Kontaktieren des Hydrierguts mit dem in gebundener Form bereitgestellten Wasserstoffs in der Transferhydrier-Einheit vorgesehen ist. Das Kontaktieren erfolgt in Gegenwart eines, insbesondere festen, Katalysators. Besonders vorteilhaft ist, wenn die wasserstoffarme Form des Hydrierguts mit der vollständig beladenen Form des flüssigen Trägermediums in Gegenwart des festen Katalysators in der Transferhydrier-Einheit kontaktiert wird. Bei einem derartigen Verfahren beträgt die mittlere Verweilzeit des Hydrierguts in der Transferhydrier-Einrichtung zwischen 30 s und 3000 min und insbesondere zwischen 10 min und 600 min. Geeignete Prozesstemperaturen, bei welchen sämtliche Reaktionsprozesse der Transferhydrierung gleichzeitig ablaufen können, liegen im Bereich zwischen 100°C und 600°C, insbesondere zwischen 250°C und 500°C und insbesondere zwischen 300°C und 450°C. Während der Reaktion stellt sich ein erhöhter Innendruck im Reaktor, insbesondere in der Transferhydrier-Einheit, ein. Der Wert des Innendrucks hängt wesentlich vom Siedepunkt des Hydrierguts ab. Der Innendruck beinhaltet insbesondere den Partialdruck am Wasserstoff, der sich temperaturabhängig und in Abhängigkeit des Mechanismus der Transferhydrierung in der Transferhydrier-Einheit ergibt.

Besonders vorteilhaft ist ein Verfahren, bei dem ein Regeln von mindestens einer Prozessgröße, insbesondere während des Hydrierens in der Transferhydrier-Einheit, mittels einer Regelungseinheit erfolgt. Das Regeln umfasst insbesondere ein Überwachen der mindestens einen Prozessgröße mittels mindestens einem mit der Regelungs-Einheit in Signalverbindung stehenden Sensor. Insbesondere dient dazu ein Temperatursensor und/oder ein Drucksensor.

Besonders vorteilhaft ist ein Verfahren, bei dem ein Trennen einer Mischung aus zumindest teilweise entladenem Trägermedium und zumindest teilweise hydriertem Hydriergut mittels einer mit der Transferhydrier-Einrichtung verbundenen Trenn-Einrichtung erfolgt. Dadurch ist es möglich, die Produkte des Verfahrens für eine weitere Handhabung effektiv zur Verfügung zu stellen. Beispielsweise kann das zumindest teilweise entladene Trägermedium einer erneuten Beladung mit Wasserstoff zugeführt werden. Das hydrierte Hydriergut wird gemäß einem vorgesehenen Prozess weiterverarbeitet.

Weitere vorteilhafte Ausgestaltungen, zusätzliche Merkmale und Einzelheiten der Erfindung ergeben sich aus der nachfolgenden Beschreibung von Ausführungsbeispielen anhand der Zeichnung. Es zeigen:

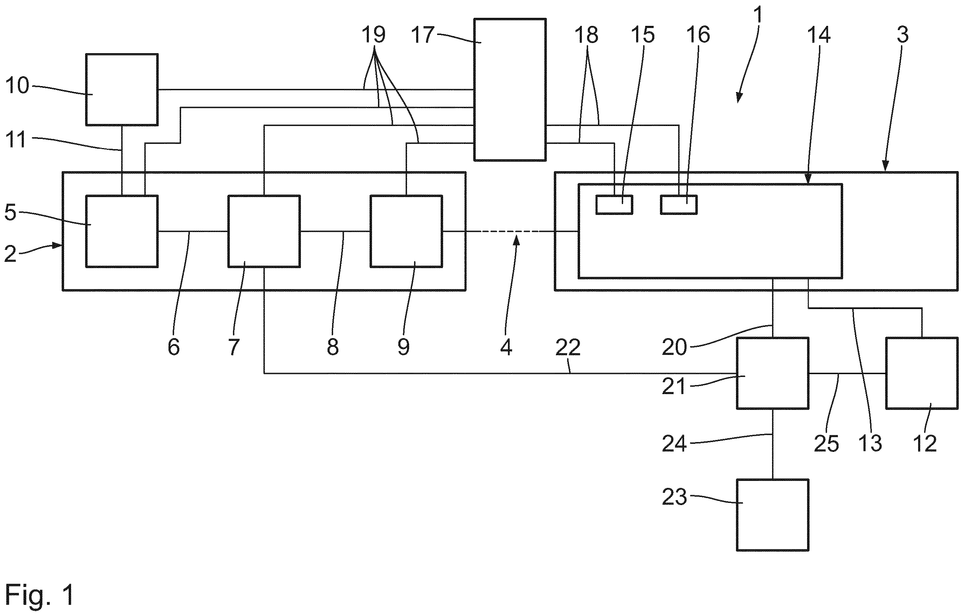

- Fig. 1: eine schematische Darstellung eines Systems zur stofflichen Nutzung von Wasserstoff.

Ein in Fig. 1 schematisch dargestelltes und als Ganzes mit 1 bezeichnetes System dient zur stofflichen Nutzung von Wasserstoff, insbesondere zur Hydrierung. Das System 1 umfasst eine Wasserstoffbereitstellungs-Einrichtung 2, die mit einer Transferhydrier-Einrichtung 3 über ein Transportmittel 4 verbunden ist. Gemäß dem gezeigten Ausführungsbeispiel ist das Transportmittel 4 durch einen Flüssigkeitstank gebildet, der beispielsweise mittels eines Schiffs transportierbar ist. Gemäß dem gezeigten Ausführungsbeispiel ist die Wasserstoffbereitstellungs-Einrichtung 2 an einem energiereichen Ort angeordnet. Die Transferhydrier-Einrichtung 3 ist an einem von dem energiereichen Ort entfernten energiearmen Ort angeordnet. Die Entfernung zwischen dem energiearmen und dem energiereichen Ort kann mehrere Tausend Kilometer betragen.

Die Wasserstoffbereitstellungs-Einrichtung 2 umfasst eine Wasserstofferzeugungs-Einheit in Form eines Elektrolyseurs 5 zum Erzeugen von Wasserstoff, insbesondere von elementarem Wasserstoff. Der Elektrolyseur 5 ist über eine Wasserstoffleitung 6 mit einer Belade-Einheit 7 verbunden. Die Belade-Einheit 7 ist Teil der Wasserstoffbereitstellungs-Einrichtung 2. Die Belade-Einheit 7 dient zum Beladen eines Trägermediums mit Wasserstoff, der im Elektrolyseur 5 aus Wasser erzeugt worden ist. Die Belade-Einheit 7 ist über eine Trägermedium-Leitung 8 mit einer Trägermediumspeicherungs-Einheit in Form eines Tanks 9 verbunden. Der Tank 9 dient zum Speichern von beladenem Trägermedium. Das Transportmittel 4 ist an den Tank 9 über eine Flüssigkeitsleitung angeschlossen. Mittels der Flüssigkeitsleitung kann beladendes Trägermedium von der Wasserstoffbereitstellungs-Einrichtung 2, insbesondere vom Tank 9, in das Transportmittel 4 und somit zu der Transferhydrier-Einrichtung 3 gefördert werden. Es ist auch denkbar, dass der Tank 9 nicht vorgesehen ist. In diesem Fall ist die Flüssigkeitsleitung direkt mit der Trägermediumleitung 8 verbunden.

Mit der Wasserstoffbereitstellungs-Einrichtung 2 ist eine Stromerzeugungs-Einrichtung 10 verbunden. Die Stromerzeugungs-Einrichtung 10 dient zur Erzeugung von elektrischem Strom, der insbesondere für die Elektrolyse im Elektrolyseur 5 erforderlich ist. Dazu ist die Stromerzeugungs-Einrichtung 10 über eine Stromleitung 11 mit dem Elektrolyseur 5 verbunden. Die Stromerzeugungs-Einrichtung 10 kann eine Einrichtung sein, die aus regenerativen Energieformen elektrischen Strom erzeugen kann. Eine Stromerzeugungs-Einrichtung 10 kann auch ein Stromleitungsnetz, also insbesondere ein öffentliches Stromleitungsnetz, sein. In das Stromleitungsnetz können Dritte Strom einspeisen und bei Bedarf entnehmen.

Die Transferhydrier-Einrichtung 3 umfasst eine Transferhydrier-Einheit 14, die zum Hydrieren eines Hydrierguts, insbesondere Toluol, dient. Das zu hydrierende Hydriergut ist beispielsweise in einem Hydriergut-Speicher 12 gelagert, der über eine Hydriergut-Zuführleitung 13 mit der Transferhydrier-Einheit 14 in Fluidverbindung steht. Über die Hydriergut-Zuführleitung 13 wird das zu hydrierende Hydriergut der Transferhydrier-Einheit 14 zugeführt.

In der Transferhydrier-Einheit 14 sind ein Temperatur-Sensor 15 zum Erfassen der Prozesstemperatur sowie ein Drucksensor 16 zum Erfassen eines Innendrucks vorgesehen. Es ist denkbar, dass weitere, insbesondere gleichartige Sensoren beispielsweise an verschiedenen Messorten innerhalb der Transferhydrier-Einheit 14 vorgesehen sind, um Messfehler zu minimieren. Es ist auch denkbar, dass weitere Sensoren vorgesehen sind, um zusätzliche Prozessgrößen zu erfassen. Um die erfassten Prozessgrößen, also die Prozesstemperatur und den Prozessdruck, darzustellen, kann eine nicht dargestellte Anzeige-Einheit vorgesehen sein, die mit den Sensoren 15, 16 direkt verbunden ist. Gemäß dem gezeigten Ausführungsbeispiel sind die Sensoren 15, 16 mit einer Regelungs-Einheit 17 über Signalleitungen 18 in bidirektionaler Signalverbindung. Die Regelungs-Einheit 17 ist über weitere Signalleitungen 19 mit Komponenten des Systems 1 jeweils in bidirektionaler Signalverbindung. Insbesondere ist die Regelungs-Einheit 17 jeweils direkt mit dem Tank 9, mit der Belade-Einheit 7, mit dem Elektrolyseur 5 und/oder mit der Stromerzeugungs-Einrichtung 10 über eine separate Signalleitung 19 verbunden.

Mit der Transferhydrier-Einrichtung 3 ist über eine Flüssigkeitsleitung 20 eine Trenn-Einrichtung 21 verbunden. Die Trenn-Einrichtung 21 dient zum Trennen einer Mischung aus zumindest teilweise entladenem Trägermedium und zumindest teilweise hydriertem Hydriergut. Die Trenn-Einrichtung 21 ist über eine Trägermedium-Leitung 22 mit der Belade-Einheit 7 verbunden. Das bedeutet, dass zumindest teilweise entladenes Trägermedium aus der Trenn-Einrichtung 21 über die Trägermedium-Leitung 22 zum erneuten Beladen in der Belade-Einheit 7 zurück gefördert werden kann. Alternativ oder zusätzlich kann beispielsweise entlang der TrägermediumLeitung 22 eine Speicher-Einheit zum Speichern des zumindest teilweise entladenen Trägermediums vorgesehen sein. Es ist auch möglich, das teilweise entladene Trägermedium aus der Trenn-Einrichtung 21 mittels geeigneter Transportmittel an einen weiteren Ort zu transportieren. In diesem Fall kann die Trägermedium-Leitung 22 entfallen.

Zum Abführen des zumindest teilweise hydrierten Hydrierguts aus der Trenn-Einrichtung 21 ist ein Hydriergut-Speicherbehälter 23 vorgesehen, der über eine Hydriergut-Leitung 24 mit der Trenn-Einrichtung 21 verbunden ist. In dem Hydriergut-Speicherbehälter 23 wird das hydrierte Hydriergut gespeichert. Nicht-hydriertes Hydriergut kann in der Trenn-Einrichtung 21 abgetrennt und über eine Hydriergut-Rückführleitung 25 dem Hydriergut-Speicher 12 rückgeführt werden.

Im Folgenden wird das erfindungsgemäße Verfahren zur stofflichen Nutzung von Wasserstoff gemäß einer ersten Ausführungsform näher erläutert. Das System 1 dient zur katalytischen Transferhydrierung. Als Hydriergut werden 0,65 mol Toluol und als Wasserstoffquelle 0,325 mol Perhydro-Mono-Benzyltoluol eingesetzt. Das Hydriergut und die Wasserstoffquelle in Form des Trägermediums werden mit jeweils 0,000325 mol eines auf Aluminiumoxid geträgerten Ruthenium-Katalysators und 0,000325 mol eines auf Kohlenstoff geträgerten PlatinKatalysators kontaktiert. Dieses Gemisch wird für 24 Stunden auf 240°C erhitzt. Dabei entwickelt sich im Reaktor ein Innendruck, zu dem der Partialdruck des Hydrierguts und ein Partialdruck an freigesetztem Wasserstoff beiträgt. Der Beitrag des Wasserstoffpartialdrucks beträgt unter diesen Bedingungen mehr als 3 bar. Nach Ende der Reaktion wird der Reaktorinhalt gekühlt und die flüssigen Produkte mittels Gaschromatographie analysiert. Die Zusammensetzung der flüssigen Produkte ergibt einen Hydriergrad des Toluols von 10,4% und einen Dehydriergrad des Trägermediums von 23,8%.

Bei dem erfindungsgemäßen Verfahren gemäß einer zweiten Ausführung werden das Hydriergut, das Trägermedium und die verwendeten Katalysatoren identisch der ersten Ausführung eingesetzt. Das so erzeugte Gemisch wird für einen vergleichsweise kurzen Zeitraum von 45 Minuten auf eine höhere Temperatur von 300°C erhitzt. Der sich in diesem Verfahren einstellende Druck, insbesondere der Beitrag des Wasserstoffpartialdrucks, beträgt unter den genannten Bedingungen mehr als 5 bar. Die Zusammensetzung der flüssigen Produkte ergibt einen Hydriergrad des Toluols von 40,8% und einen Dehydriergrad des Trägermediums von 53,8%.

Das erfindungsgemäße Verfahren gemäß einem dritten Ausführungsbeispiel basiert auf den identischen Einsatzstoffen gemäß dem ersten Ausführungsbeispiel. Gegenüber dem ersten Ausführungsbeispiel beträgt die Erwärmungsdauer lediglich 30 Minuten. Die Erwärmungstemperatur beträgt 350°C. Der Beitrag des Wasserstoffpartialdrucks beträgt mehr als 5 bar. Die Zusammensetzung der flüssigen Produkte ergibt einen Hydriergrad des Toluols von 95,5% und einen Dehydriergrad des Trägermediums von 99,2%.

## Patentansprüche

1. Verfahren zur stofflichen Nutzung von Wasserstoff umfassend die Verfahrensschritte
- Beladen eines Trägermediums mit Wasserstoff mittels einer Belade-Einheit (7),
- Bereitstellen von dem an dem Trägermedium gebundenen Wasserstoff mittels einer Wasserstoffbereitstellungs-Einrichtung (2),
- Hydrieren eines Hydrierguts unter Verwendung des in gebundener Form als flüssiges, wasserstoffbeladenes Trägermedium bereitgestellten Wasserstoffs mittels einer Transferhydrier-Einheit (14) einer Transferhydrier-Einrichtung (3), wobei das Hydriergut durch direktes Kontaktieren mit dem flüssigen, wasserstoffbeladenen Trägermedium hydriert wird, so dass die Handhabung und Lagerung von molekularem Wasserstoff in der Transferhydrier-Einrichtung (3) vermieden ist.

2. Verfahren gemäß Anspruch 1, **gekennzeichnet durch** ein Kontaktieren des Hydrierguts mit dem in gebundener Form bereitgestellten Wasserstoff in Gegenwart eines Katalysators in der Transferhydrier-Einheit (14).

3. Verfahren gemäß Anspruch 1 oder 2, **gekennzeichnet durch** ein Regeln mindestens einer Prozessgröße, insbesondere während des Hydrierens in der Transferhydrier-Einheit (14), mittels einer Regelungs-Einheit (17), wobei das Regeln insbesondere ein Überwachen der mindestens einen Prozessgrößen mittels mindestens eines mit der Regelungs-Einheit (17) in Signalverbindung stehenden Sensors, insbesondere eines Temperatursensors (15) und/oder eines Drucksensors (16), umfasst.

4. Verfahren gemäß einem der Ansprüche 1 bis 3, **gekennzeichnet durch** ein Trennen einer Mischung aus zumindest teilweise entladenem Trägermedium und zumindest teilweise hydriertem Hydriergut mittels einer mit der Transferhydrier-Einrichtung (3) verbundenen Trenn-Einrichtung (21).

## Claims

1. Method for the material use of hydrogen comprising the method steps
- Loading of a carrier medium with hydrogen by means of a loading unit (7),
- Provision of the hydrogen bound to the carrier medium by means of hydrogen-provision device (2),
- Hydrogenation of a material for hydrogenation with use of the hydrogen provided in bound form as a liquid, hydrogen-loaded carrier medium by means of a transfer-hydrogenation unit (4) of a transfer-hydrogenation unit (3), wherein the material for hydrogenation is hydrated by direct contact with the liquid, hydrogen-loaded carrier medium such that the handling and storage of molecular hydrogen in the transfer-hydrogenation unit (3) is dispensed with.

2. Method according to claim 1, **characterized by** a contacting of the material for hydrogenation with the hydrogen provided in bound form in the presence of a catalyst in the transfer-hydrogenation unit (14).

3. Method according to claim 1 or 2, **characterized by** a control of at least one process parameter, especially during the hydrogenation in the transfer-hydrogenation unit (14), by means of a control unit (17), wherein the control comprises especially a monitoring of the at least one process parameter by means of at least one sensor disposed in signal connection with the control unit (17), especially a temperature sensor (15) and/or a pressure sensor (16).

4. Method according to one of claims 1 to 3, **characterized by** a separation of a mixture of at least partially unloaded carrier medium and at least partially hydrogenated material for hydrogenation by means of a separating device (21) connected with the transfer-hydrogenation facility (3).

## Revendications

1. Procédé d'utilisation matérielle d'hydrogène, comprenant les étapes de procédé
- de chargement d'un milieu porteur en hydrogène au moyen d'une unité de chargement (7),
- de préparation de l'hydrogène lié au milieu porteur au moyen d'un dispositif de préparation d'hydrogène (2),
- d'hydrogénation d'un produit à hydrogéner en utilisant l'hydrogène préparé sous une forme liée en tant que milieu porteur liquide chargé en hydrogène au moyen d'une unité d'hydrogénation par transfert (14) d'un dispositif d'hydrogénation par transfert (3), dans lequel le produit à hydrogéner est hydrogéné par contact direct avec le milieu porteur liquide, chargé en hydrogène de sorte que la manutention et le stockage d'hydrogène moléculaire sont évités dans le dispositif d'hydrogénation par transfert (3).

2. Procédé selon la revendication 1, **caractérisé par** une mise en contact du produit à hydrogéner avec l'hydrogène préparé sous une forme liée en présence d'un catalyseur dans l'unité d'hydrogénation par transfert (14) .

3. Procédé selon la revendication 1 ou 2, **caractérisé par** une régulation d'au moins une grandeur de processus, en particulier au cours de l'hydrogénation dans l'unité d'hydrogénation par transfert (14), au moyen d'une unité de régulation (17), dans lequel la régulation comprend en particulier une surveillance de l'au moins une grandeur de processus au moyen d'au moins un capteur en liaison de signalisation avec l'unité de régulation (17), en particulier d'un capteur de température (15) et/ou d'un capteur de pression (16).

4. Procédé selon l'une quelconque des revendications 1 à 3, **caractérisé par** une séparation d'un mélange composé d'un milieu porteur au moins en partie déchargé et d'un produit à hydrogéner au moins en partie hydrogéné au moyen d'un dispositif de séparation (21) relié au dispositif d'hydrogénation par transfert (3) .
